# EUROPEAN PATENT SPECIFICATION

(11) **EP 2 999 717 B1**
(45) Date of publication and mention of the grant of the patent: **08.08.2018**
(21) Application number: 14736034.1
(22) Date of filing: 21.05.2014
(51) Int. Cl.: C07K 16/28, C07K 16/42, A61P 35/00

(54) **TREATMENT OF MAST CELL RELATED PATHOLOGIES**
BEHANDLUNG VON MASTZELLEN-VERMITTELTEN ERKRANKUNGEN
TRAITEMENT DES PATHOLOGIES IMPLIQUANT DES MASTOCYTES

(30) Priority: 21.05.2013 US 201361825807 P
(43) Date of publication of application: 30.03.2016
(73) Proprietor: Yissum Research Development Company of the Hebrew University of Jerusalem Ltd., 9139002 Jerusalem (IL)
(72) Inventor: LEVI-SCHAFFER, Francesca, 9314915 Jerusalem (IL); MIZRAHI, Saar, 9985500 Doar-Na HaEla (IL); BEN-ZIMRA, Micha, 9230618 Jerusalem (IL); KARRA, Laila, 6806101 Jaffa (IL)
(74) Representative: Dennemeyer & Associates S.A.
(86) International application number: PCT/IL2014/050449
(87) International publication number: WO 2014/188423

(56) References cited:
- CHRISTOPHER J SCOTT ET AL: "Immunocolloidal Targeting of the Endocytotic Siglec-7 Receptor Using Peripheral Attachment of Siglec-7 Antibodies to Poly(Lactide-co-Glycolide) Nanoparticles", PHARMACEUTICAL RESEARCH, KLUWER ACADEMIC PUBLISHERS-PLENUM PUBLISHERS, NL, vol. 25, no. 1, 3 August 2007 (2007-08-03) , pages 135-146, XP019579460, ISSN: 1573-904X
- ITO<A B C> A ET AL: "Binding specificity of siglec7 to disialogangliosides of renal cell carcinoma: possible role of disialogangliosides in tumor progression", FEBS LETTERS, ELSEVIER, AMSTERDAM, NL, vol. 504, no. 1-2, 24 August 2001 (2001-08-24), pages 82-86, XP004300912, ISSN: 0014-5793, DOI: 10.1016/S0014-5793(01)02734-X
- FALCO M ET AL: "Identification and molecular cloning of p75/AIRM1, a novel member of the sialoadhesin family that functions as an inhibitory receptor in human natural killer cells.", THE JOURNAL OF EXPERIMENTAL MEDICINE 20 SEP 1999, vol. 190, no. 6, 20 September 1999 (1999-09-20), pages 793-802, XP002729943, ISSN: 0022-1007
- NICOLL GAVIN ET AL: "Ganglioside GD3 expression on target cells can modulate NK cell cytotoxicity via siglec-7-dependent and -independent mechanisms.", EUROPEAN JOURNAL OF IMMUNOLOGY JUN 2003, vol. 33, no. 6, June 2003 (2003-06), pages 1642-1648, XP002729944, ISSN: 0014-2980
- VITALE CHIARA ET AL: "Engagement of p75 / AIRM1 or CD33 inhibits the proliferation of normal or leukemic myeloid cells", PROCEEDINGS OF THE NATIONAL ACADEMY OF SCIENCES, NATIONAL ACADEMY OF SCIENCES, vol. 96, no. 26, 21 December 1999 (1999-12-21), pages 15091-15096, XP002171141, ISSN: 0027-8424, DOI: 10.1073/PNAS.96.26.15091
- BACHELET ET AL: "Abrogation of allergic reactions by a bispecific antibody fragment linking IgE to CD300a", JOURNAL OF ALLERGY AND CLINICAL IMMUNOLOGY, ELSEVIER, AMSTERDAM, NL, vol. 117, no. 6, 1 June 2006 (2006-06-01), pages 1314-1320, XP005513526, ISSN: 0091-6749, DOI: 10.1016/J.JACI.2006.04.031
- MIZRAHI SA'AR ET AL: "Siglec-7 is an inhibitory receptor on human mast cells and basophils.", THE JOURNAL OF ALLERGY AND CLINICAL IMMUNOLOGY JUL 2014, vol. 134, no. 1, July 2014 (2014-07), pages 230-233.e3, XP002729945, ISSN: 1097-6825

## Description

### FIELD AND BACKGROUND OF THE INVENTION

The present disclosure in some embodiments thereof, relates to compositions for and methods of inhibiting activation of a mast cell and treating a disease induced by activation of a mast cell.

Mast cells (MCs) are highly granulated cells widely distributed throughout the body. MCs are best known for their role in allergic diseases such as asthma, allergic rhinitis, allergic conjunctivitis, and atopic dermatitis. Among these diseases, and as an example, asthma is the most common illness of early childhood, counting for up to 20 % in Western countries [Busse et al. (2001) N Engl J Med. 344:3501]. MCs are able to bind common allergens such as flower pollens and house dust mite and to subsequently undergo activation and secrete granules containing an array of mediators such as histamine, that cause symptoms such as skin irritation, running nose or shortness of breath. MCs are also indicated in hypersensitivity and cancer such as systemic mastocytosis (SM).

The classical activation of MCs is triggered by the binding of allergens to two adjacent IgE molecules bound to FcεRI, however there are other IgE-independent ways of MCs stimulation. It has recently become clear that MCs degranulation is regulated by additional activatory and inhibitory surface receptors such as KIR, IRP60, FcyRIIB and gp49A/B 1/B2.

The inhibitory receptors were first discovered on natural killer (NK) cells and in recent years on other cells of the immune system, including MCs and eosinophils (Eos). When inhibitory receptors are engaged they deliver signals into the cell that inhibit different functions, such as activation, cell proliferation, survival and differentiation. It has been shown that the NK cells inhibitory receptor, CD300a (IRp60) is also found on MCs and Eos and can inhibit their function when activated by cross-linking it to an activating receptor present on the cell surface [e.g., Munitz et al. Blood. (2006) 107(5): 1996-2003; Bachelet et al. J Immunol. (2008) 180(9):6064-9; Bachelet et al. J. Allergy Clin. Immunol. (2006)117(6):1314-20.; and Bachelet et al. J. Immunol. (2005)175(12):7989-95).

Sialic acid-binding immunoglobulin superfamily lectins (Siglecs) are a family of trans-membrane proteins differentially expressed on hematopoietic cells that bind as ligands sialic acid containing carbohydrate groups of glycoproteins and glycolipids. Cell surface glycosylation has important regulatory functions in the maturation, activation, and homeostasis of immune cells and the Siglec family of proteins transmit inhibitory as well as activating signals intimately involved in the regulation of immune responses.

Siglec-7, a type 1 trans-membrane protein first cloned in 1999 belongs to the human Siglec family of receptors, is characterized by a sialic acid binding N-terminal V-set Ig domain, two C2-set Ig domains and an intracytoplasmic region containing one immune-receptor tyrosine based inhibitory motif (ITIM) and one ITIM-like motif. Siglec-7 is known to be constitutively expressed on natural killer (NK) cells, monocytes and on a small subset of CD8⁺CD3⁺ T cells. Siglec-7 has generally been indicated as transmitting inhibitory signals, however targeting of Siglec-7 has different effects on cell as previously reported, for example:
Use of anti-Siglec-7 specific monoclonal antibody (mAb) induced activation of monocytes [Varchetta et al. PLoS One. 2012; 7(9): e45821.];
use of an anti-Siglec F(ab')(2) induced non-apoptotic monocytes' cell death in an ITIM independent manner [Mitsuki et al. (2010) Glycobiology 20(3):395-402];
and use of anti-Siglec-7 mAb inhibited the proliferation of chronic and acute myeloid leukemia (Vitale et al. (1999) Proc Natl Acad Sci USA. 96(26):15091-6; and Vitale et al. (2001) Proc Natl Acad Sci U S A 98(10):5764-9).

On the other hand it was shown that Siglec-7 can contribute to the development of cancer by inhibiting the activity of NK cells which play a major role in anti-tumor immune surveillance. For example, it has been shown that renal cell carcinoma (RCC) expressing high levels of the ganglioside DSGb5, a preferred ligand for Siglec-7, were protected form lysis by NK cells compared to cells expressing low levels of DSGb5 (Kawasaki et al. (2010) Glycobiology. 20(11):1373-9).

### ADDITIONAL RELATED ART

Yokoi et al. Allergy (2006) 61:769-76;
U.S. Publication No. 20020110862;
U.S. Patent No. 8133485;
Ott and Cambier; J. Allergy Clin. Immunol. (2000) 429-440;
International Publication No: WO03064662;
International Publication No: WO03030835

### SUMMARY OF THE INVENTION

The present invention is defined in the appended claims. Preferred embodiments are set out in the subclaims and in this specification.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

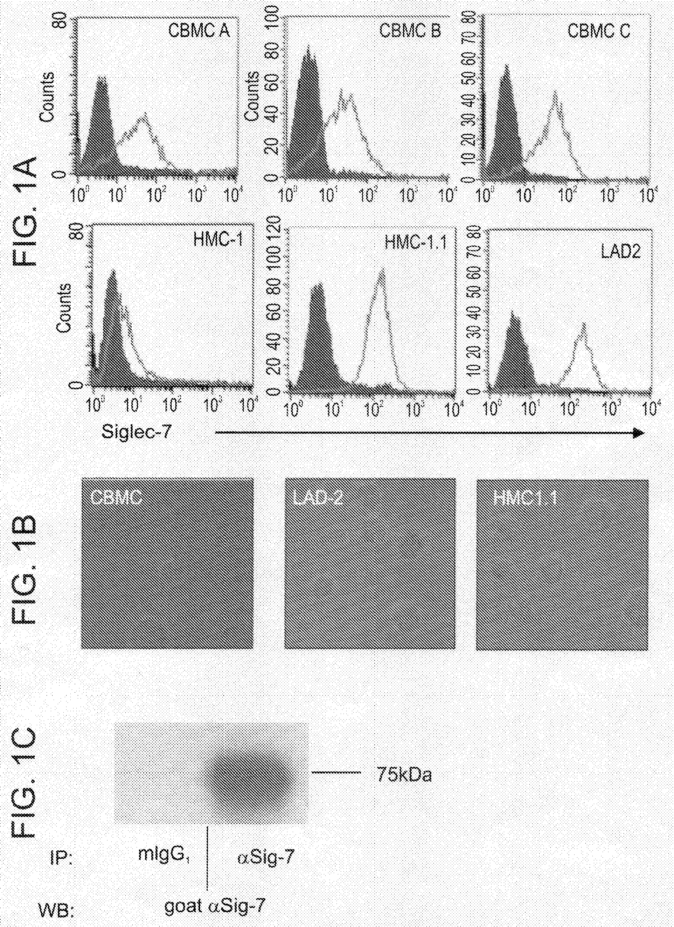

FIGs. 1A-C demonstrate positive expression of Siglec-7 in mast cells (MCs). Figure 1A shows flow cytometry histograms of human cord-blood mast cells obtained from three donors (CBMC-A, CBMC-B and CBMC-C) and three human MCs tumor lines (HMC-1, HMC1.1 and LAD-2). Cells were stained with mouse anti-human Siglec-7 antibody followed by FITC-conjugated goat F(ab')2 anti-mouse IgG antibody (green histograms). Matched mouse IgG1 (mIgG1) isotype controls are shown in the respective purple histograms. Figure 1B shows representative immunofluorescence photomicrographs (magnification x 630) of CBMCs and human MCs tumor lines stained with mouse anti-human Siglec-7 antibody followed by goat anti-mouse Alexa Flour® 647 as secondary antibody. Figure 1C is a representative western blot photograph demonstrating expression of Siglec-7 protein in CBMCs. Right lane shows positive expression of Siglec-7 in lysed CBMCs immunoprecipitated (IP) with mouse anti-human Siglec-7 antibody followed by western blot (WB) with goat anti-human Siglec-7 and anti-goat-HRP as secondary. Left lane show negative expression of Siglec-7 protein in WB of a sample from control IP using mIgG1 isotype control antibody.

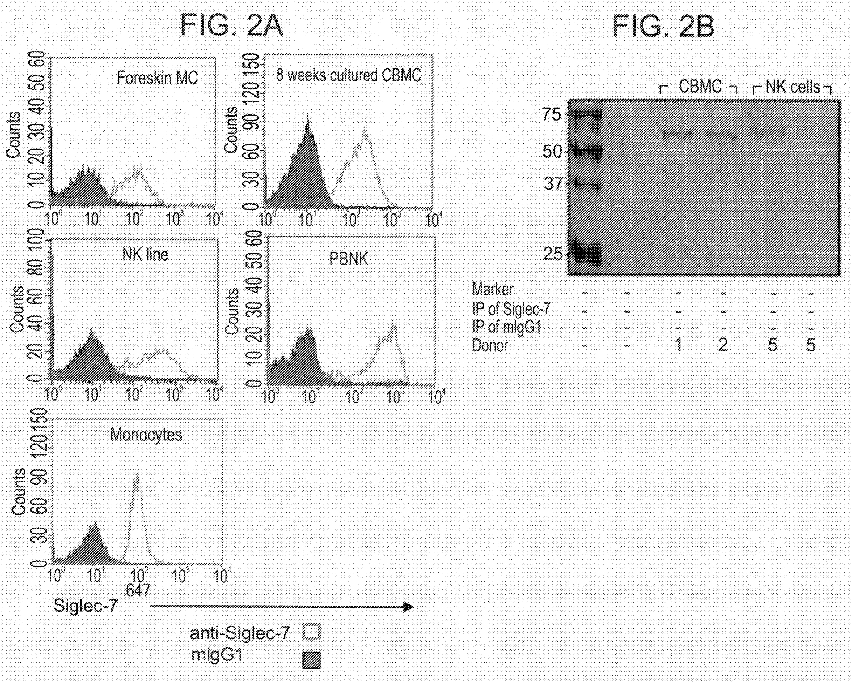

FIGs. 2A-B demonstrate expression of Siglec-7 in human MCs, NKs, and monocytes. Figure 2A shows representative flow cytometry histograms of human foreskin MCs, CBMCs, NK line, peripheral blood NK cells and monocytes. Cells were stained with mouse anti-human Siglec-7 antibody followed by FITC-conjugated goat F(ab')2 anti-mouse IgG antibody (green histograms). Matched mIgG1 isotype controls are shown in the respective purple histograms. Figure 2B is a representative western blot photograph demonstrating positive expression of Siglec-7 protein in lysed CBMCs and NKs cell line immunoprecipitated (IP) with mouse anti-human Siglec-7 antibody followed by WB with goat anti-human Siglec-7 and anti-goat-HRP as secondary. Second lane from the left is a negative control with no lysate.

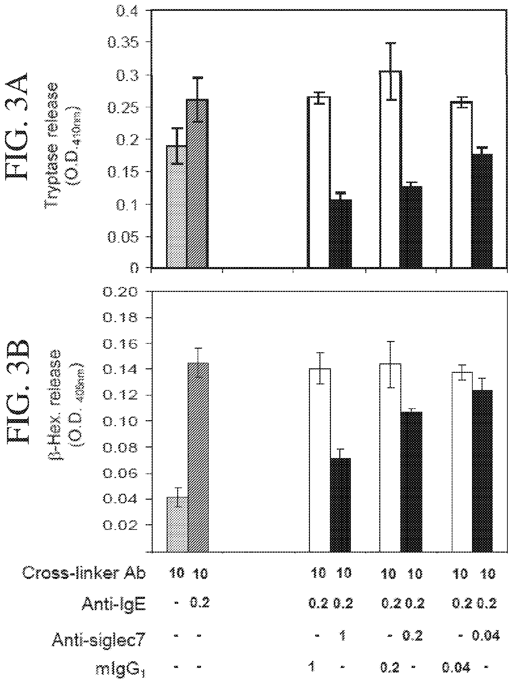

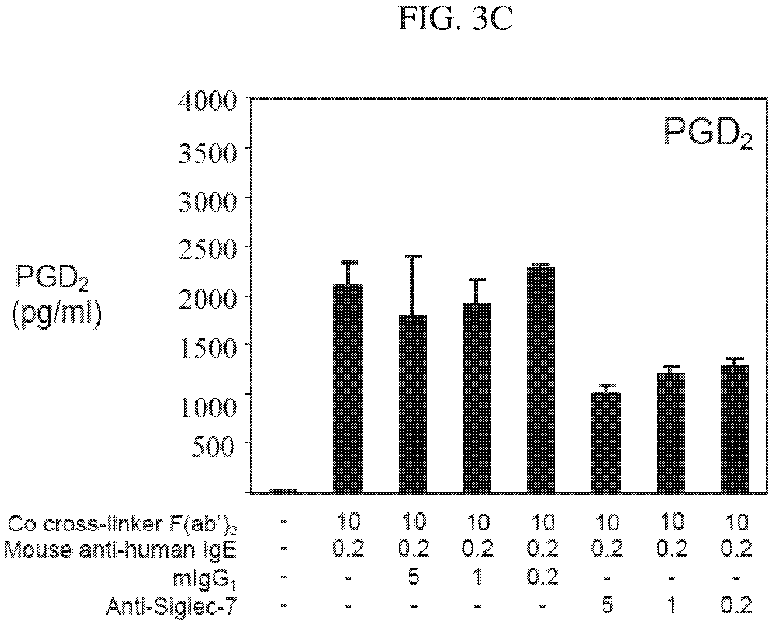

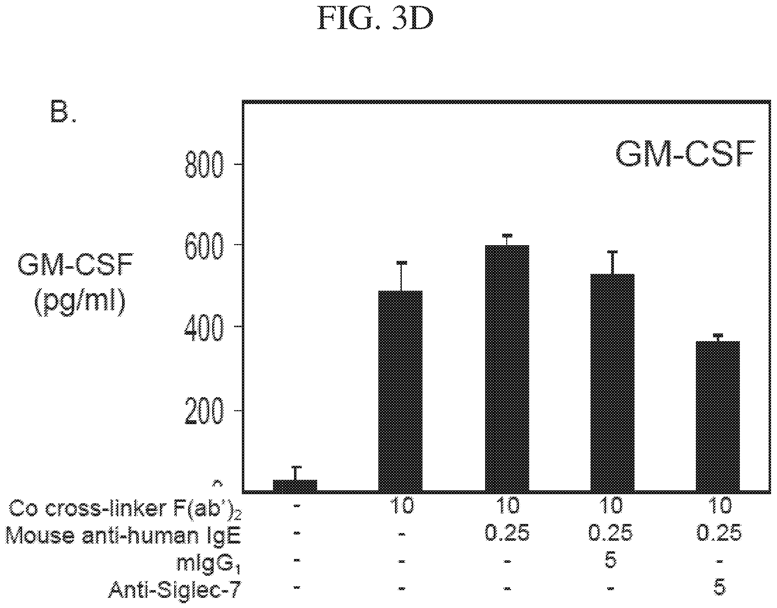

FIGs. 3A-D are graphs demonstrating the effect of Siglec-7 stimulation on IgE-dependent mediator release from CBMCs. Release of tryptase (Figure 3A), β-hexosaminidase (β-hex, Figure 3B), PGD2 (Figure 3C) and GM-CSF (Figure 3D) by the cells to the culture medium are shown. Data are presented as the mean ± SEM of triplicates from 1 experiment. Antibodies concentrations are in µg / ml.

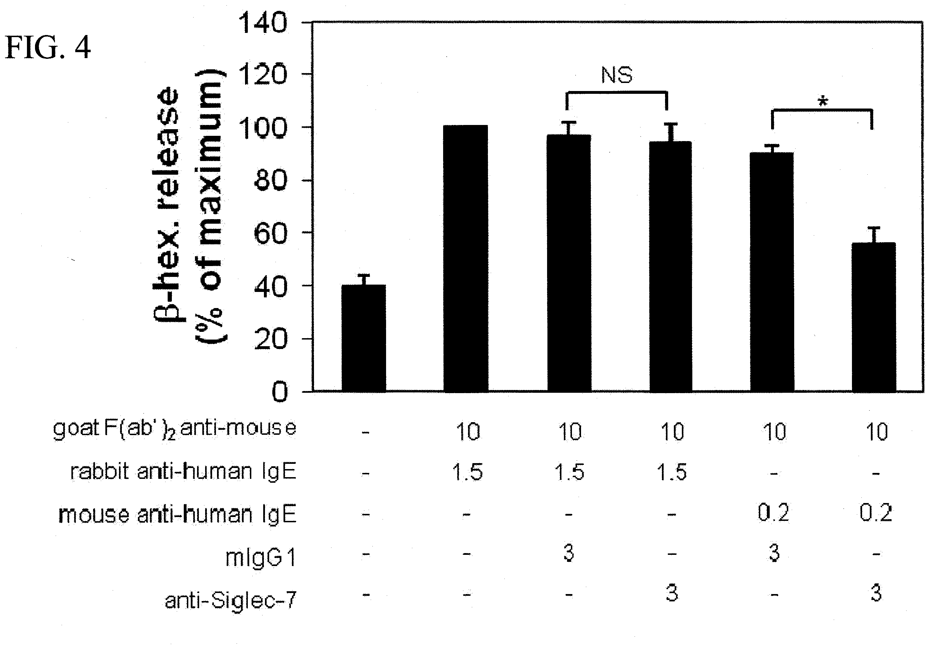

FIG. 4 is a graph demonstrating the effect of Siglec-7 stimulation on IgE-dependent β-hex release from CBMCs without co-cross-linking. Data is presented as the mean ± SEM from 2 separate experiments. Antibodies concentrations are in µg / ml. *P ≤ 0.03.

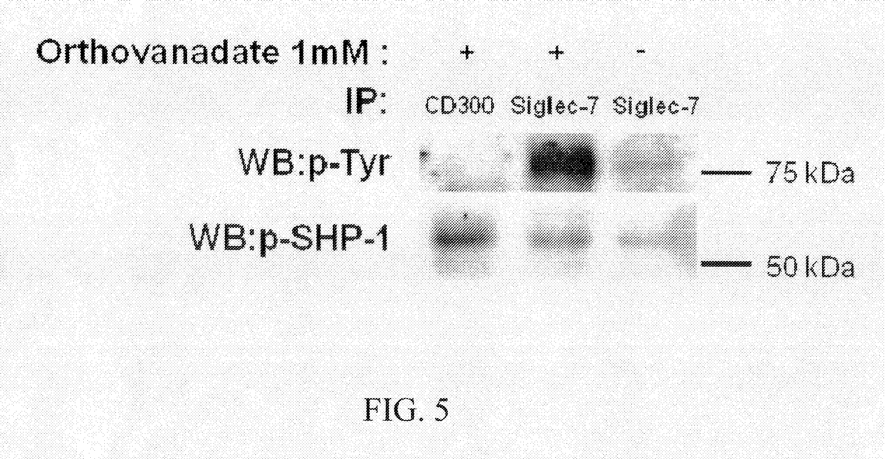

FIG. 5 is a representative WB photograph demonstrating co-immunopercipitation of phosphorylated SHP-1 with Siglec-7 and CD300a in CBMCs incubated with or without orthovanadate.

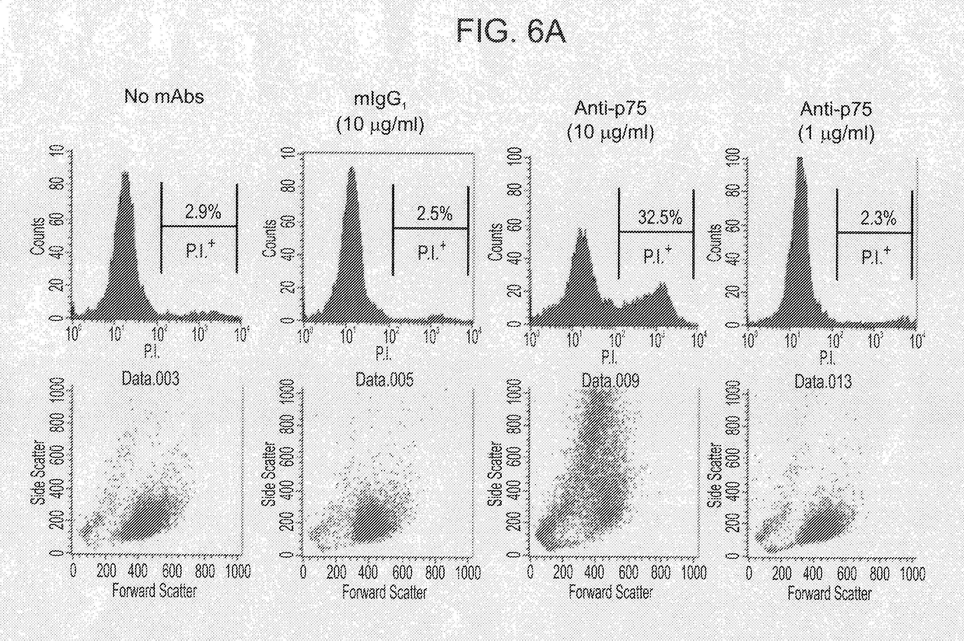

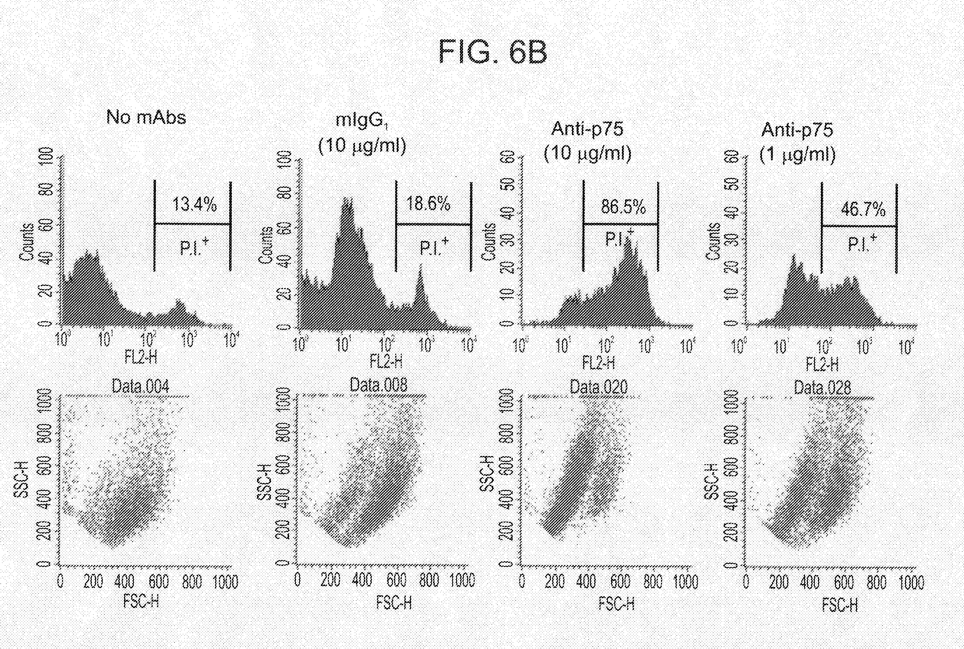

FIGs. 6A-B are representative flow cytometry plots demonstrating cell death of tumor MCs in response to Siglec-7 activation. HMC-1 cells (Figure 6A) and LAD-1 cells (Figure 6B) were incubated with mouse anti-human Siglec-7 mAb or mIgG isotype control, cross linked with F(ab')2 goat anti-mouse and stained with PI.

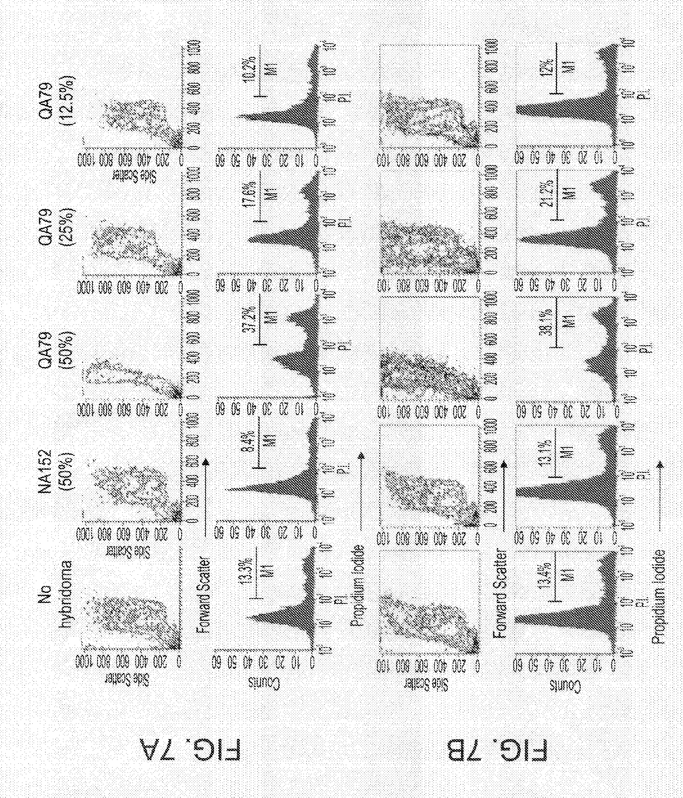

FIGs. 7A-B are representative flow cytometry plots demonstrating cell death of tumor MCs in response to Siglec-7 activation. HMC-1.1 cells were incubated with mouse anti-human Siglec-7 hybridoma's sup (QA79) or non-relevant hybridoma's sup (NA152), and then either cross-linked with F(ab')2 goat anti-mouse (Figure 7A) or without cross-linker (Figure 7B) and stained with PI.

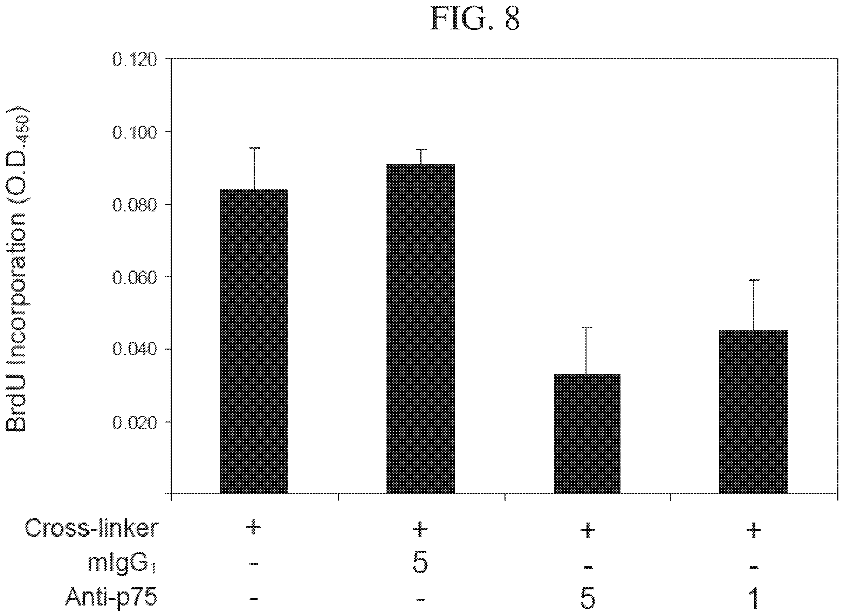

FIG. 8 is a graph showing the effect of Siglec-7 activation on proliferation of mast tumor cells as evaluated by BrdU incorporation. The level of BrdU uptake was determined in HMC-1.1 cells that were incubated with mouse anti-human Siglec-7 mAb or mIgG1 isotype control and cross-linked with F(ab')2 goat anti-mouse.

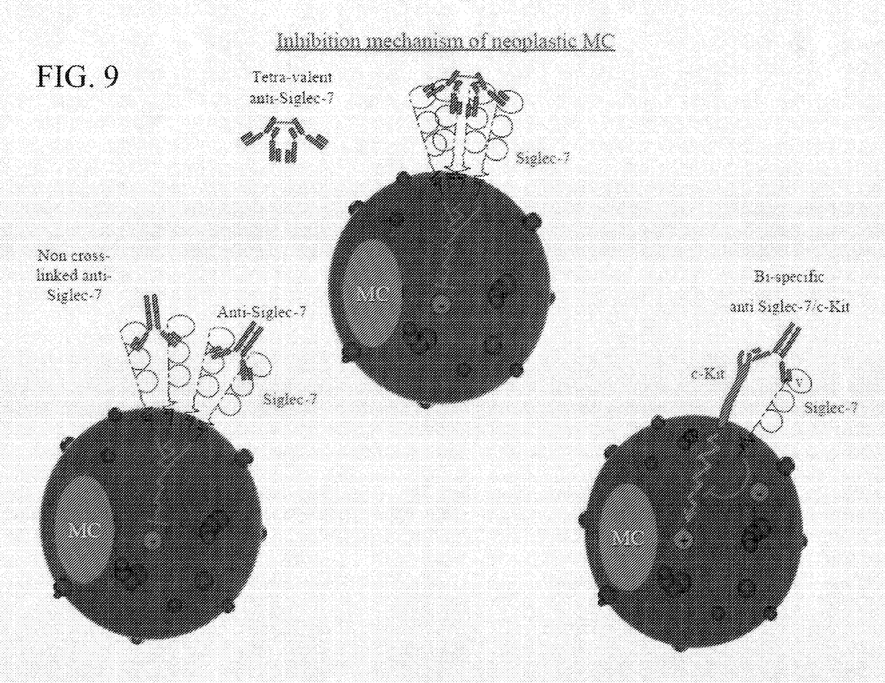

FIG. 9 is a schematic representation of Siglec inhibitory mechanism of action on tumor mast cells.

### DESCRIPTION OF SPECIFIC EMBODIMENTS OF THE INVENTION

The present invention generally relates to compositions for and methods of inhibiting activation of a mast cell and treating a disease induced by activation of a mast cell.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Mast cells (MCs) are highly granulated cells widely distributed throughout the body. Classical activation of MCs is triggered by the binding of allergens to two adjacent IgE molecules bound to FcεRI, however there are other IgE-independent ways for MCs stimulation. It has recently become clear that MCs degranulation is regulated by additional activating and inhibitory surface receptors such as KIR, IRP60, FcyRIIB and gp49A/Bl/B2.

Siglec-7, a type 1 trans-membrane protein that belongs to the human Siglec family of receptors, is known to be constitutively expressed on natural killer (NK) cells, monocytes and on a small subset of CD8⁺CD3⁺ T cells. Siglec-7 has generally been indicated as transmitting inhibitory signals, however targeting of Siglec-7 was also shown to induce activation of monocytes, to induce monocytes' cell death and to inhibit the proliferation of chronic and acute myeloid leukemia.

Whilst reducing the present invention to practice the present inventors have surprisingly uncovered that Siglec-7 is expressed on the surface of normal and tumorigenic MCs. Indeed Siglec-7 was found to function on the surface of these cells by eliciting inhibitory signaling. The present inventors have therefore harnessed these new findings towards configuring a novel tool for MC activation inhibition.

Thus, while further reducing the present invention to practice, the present inventors have shown that co-cross-linking of the inhibitory receptor Siglec-7 with an activation receptor inhibits degranulation of non-cancerous MCs without reducing their viability. In addition, cross-linking of Siglec-7 increases the mortality of transformed MCs and inhibits their proliferation.

Taken together, according to this invention, an agent that activates Siglec-7 can be used to reduce viability of cancerous MCs. In addition, according to this invention an agent that binds Siglec-7 while cross linking it to a MC activating receptor can be used to inhibit activation of MCs.

As is illustrated hereinunder and in the Examples section, which follows the present inventors have shown that Siglec-7 is expressed on the surface of normal human cord blood MCs, isolated human foreskin MCs and neoplastic MC lines such as HMC-1, HMC-1.1 and LAD (Example 1, Figures 1A-B and 2A). Furthermore, as demonstrated in Figures 1C and 2B (Example 1), MC-associated Siglec-7 is highly glycosylated.

As is shown in Figures 3A-D, Example 2, activating Siglec-7 with mouse anti-Siglec-7 mAb and co-cross linking Siglec-7 with the activation signal of FcεRI using a mouse anti IgE mAb followed by goat F(ab')2 anti mouse IgG significantly reduced the secretion of tryptase, β-hexosaminidase, PGD2 and GM-CSF in a dose dependent manner; suggesting that Siglec-7 functions as an inhibitory receptor on normal human MCs capable of inhibiting the release of mediators belonging to all three major groups of mediators released by activated MC.

The inventors further demonstrate that cross-linking Siglec-7 alone did not inhibit FcεRI-induced degranulation (Figure 4, Example 2), suggesting that Siglec-7 must be coupled to FcεRI by co-cross-linking to effectively inhibit mediator release from CBMCs. It is also suggested that intercellular interactions that occur during allergic inflammation can induce robust Siglec-7 clustering strong enough to induce self-activation without the requirement for co-cross-linking with an activating receptor.

Additionally, tyrosine-phosphorylated SHP-1 co- immnunoprecipitated with Siglec-7 in cord blood MCs (Figure 5, Example 2) indicating a possible role for SHP-1 in mediating the signals downstream of activated Siglec-7 in cord blood MCs.

The present inventors have further demonstrated in Figures 6A-B, 7A-B and 8, Example 3, that cross-linking of Siglec-7 with anti-Siglec-7 mAb reduced proliferation and caused death of MC tumorigenic cell lines. Thus indicating that cross-linking of Siglec-7 affects the viability and proliferative capacity of tumorigenic MCs.

Consequently, the present invention describes agents and compositions that binds and activates Siglec-7 and can be used to inhibit activation of MCs and reduce viability of cancerous MCs *in-vitro, ex-vivo* and *in-vivo.* Furthermore, the agents and composition can be used in the treatment of medical conditions induced by activation of MCs.

Thus, according to an aspect, there is provided a method of inhibiting activation of a cancerous MC, the method comprising contacting the cancerous MC with an effective amount of a multivalent agent which binds and activates Siglec-7, thereby inhibiting activation of the cancerous MC.

According to an additional or an alternative embodiment, there is provided a method of inhibiting activation of a MC, the method comprising contacting a MC with an effective amount of an agent comprising:
(i) a first moiety which binds and activates Siglec-7; and
(ii) a second moiety which binds activates a MC activating receptor,
thereby inhibiting activation of the mast cell.

According to an additional or an alternative embodiment, there is provided a method of inhibiting activation of a MC, the method comprising contacting the MC, with an effective amount of a multivalent agent which binds and activates Siglec-7, thereby inhibiting activation of the mast cell.

As used herein "a mast cell (MC)" refers to a highly granulated cell containing numerous granules comprising substances such as histamine and heparin. Typical markers of MCs include but are not limited to CD117 (c-Kit) and FcεRI.

Activation of mast cells is defined by at least one of the following processes growth, maturation, proliferation, migration, survival, apoptosis, degranulation, mediator release, priming (preparing the cell for action, alerting it to standby), chemotaxis, adherence, and synthesis of cytokines, growth factors, arachidonic acid metabolites, chemokines, phospholipid metabolites and others.

As mentioned the MC can be cancerous or non-cancerous.

According to a specific embodiment the MC can be activated.

According to an embodiment, an activated MC is physiologically (e.g., in vivo) activated cell. Alternatively, the activated cell is or an artificially induced cell such as by the use of an anti-IgE antibody.

According to a specific alternate embodiment, the MC is non-activated.

According to specific embodiments activation is selected from the group consisting of proliferation, survival and secretion of mediators.

According to specific embodiments, the activation is IgE dependent.

According to specific embodiments, the activation is not dependent on IgE.

As used herein, the term "secretion of mediators" refers to degranulation and release of a pre-formed and/or de-novo synthesized metabolites from a MC (e.g. such as histamine, tryptase, proteoglycans, beta-hexosaminidase, PGD2, and an array of cytokines and growth factors, such as further detailed hereinbelow. Upon activation of a MC (e.g. by binding of an allergen to IgE attached to the surface of a MC, or artificial activation), a variety of mediators are released which give rise to increased vascular permeation, vasodilation, bronchial and visceral smooth muscle contraction, and local inflammation. In the most extreme form of immediate hypersensitivity reaction known as anaphylaxis, mediators released from MCs can restrict airways to the point of asphyxiation.

Generally, Mediators released from MCs may be divided into two broad classes, pre-formed or secretory granule associated mediators and non-pre-formed or newly synthesized mediators. The pre-formed mediators include biogenic amines, most notably histamine. The pre-formed mediators also comprise granule macromolecules such as proteoglycans, most notably heparin and chondroitin sulfate E; chemotactic factors such as eosinophil and neutrophil chemotactic factors of anaphylaxis; and enzymes such as proteases, tryptase, beta-hexosaminidase, chymase, cathepsin G-like enzyme, elastase, carboxypeptidase A and acid hydrolases. The non-pre-formed mediators or newly synthesized mediators include products of arachidonic acids such as but not limited to leukotrienes, prostaglandins, thromboxanes and platelet activating factor (PAF). Some examples of leukotrienes include LTC4, LTD4, LTE4, and LTB4. Examples of prostaglandins and thromboxanes include prostaglandin D₂ (PGD2), prostaglandin F2 (PGF2) and thromboxane A2 (TXA2). Cytokines including chemokines are another class of mediators. These cytokines are predominantly responsible for the late phase reaction which begins two to four hours after elicitation of many immediate hypersensitivity reactions. Some examples of cytokines include but are not limited to interleukin-lb (IL-lb), IL-3, IL-5, GM-CSF, and tumor necrosis factor alpha (TNF-α). Examples of chemokines include MCP-1, miplb, IL-8, and RANTES.

Methods of monitoring activation and/or inhibition of activation (also referred to as state of activation) of a MC are known in the art and include for example, assays which evaluate cell viability and survival such as the MTT test which is based on the selective ability of living cells to reduce the yellow salt MTT (3-(4, 5-dimethylthiazolyl-2)-2, 5-diphenyltetrazolium bromide) (Sigma, Aldrich St Louis, MO, USA) to a purple-blue insoluble formazan precipitate; the Annexin V assay [ApoAlert® Annexin V Apoptosis Kit (Clontech Laboratories, Inc., CA, USA)]; the Senescence associated-P-galactosidase assay (Dimri GP, Lee X, et al. 1995. A biomarker that identifies senescent human cells in culture and in aging skin in vivo. Proc Natl Acad Sci USA 92:9363-9367); the TUNEL assay [Roche, Mannheim, Germany]; Assays which evaluate cell proliferation capacity such as the BrdU incorporation assay [Cell Proliferation ELISA BrdU colorimetric kit (Roche, Mannheim, Germany)]; assays which evaluate secretion of mediators such as ELISA (e.g. PGD₂ ELISA kit (Cayman chemicals); GM-CSF ELISA kit (Peprotech); colorimetric and fluorometric enzymatic assays based on incubation with the specific mediator's susbstrate (e.g. Bachelet et al. J. Immunol. (2005) 175:7989-95; and assay [Gibbs et al. Clin Exp Allergy (2008) 38:480-5]; As well as various RNA and protein detection methods, such as evaluating the level of phosphorylation on tyrosine residues on key signal molecules such as the kinases syk, lyn,fyn, erk. The higher the level of phosphorylation, the higher the MC activation is. This can be detected by flow cytometry analysis or by Western blot analysis using anti specific anti-phosphotyrosine antibodies for the same molecules.

As used herein the term "inhibiting activation" refers to a statistically significant decrease in activation, e.g., as defined hereinabove, as compared to a control cell (the respective mast cell) being under the same assay conditions without the treatment with the agent.

According to specific embodiments inhibiting activation refers both to suppressing activation and to preventing activation.

According to a specific embodiment, inhibiting activation is by at least 10 %, 20 %, 30 %, 50 %, 80 %, 90 %, 95 % and even 100 % as compared to the activation in the control cell.

As used herein, the term "contacting" refers to bringing the agent to direct contact with an MC. It is contemplated both applying the agents described herein to an isolated MC (e.g., a primary cell culture, a cell line), to a biological sample or tissue comprising same (e.g., a blood sample, skin tissue) or to an organism (e.g., human subject).

Thus, the contacting can be effected *in-vitro, ex-vivo* or *in-vivo* (the latter is also referred to as administering).

According to specific embodiments the contacting is effected *ex-vivo* such as by incubating a biological sample comprising the cells with the agent.

As used herein, an "effective amount" refers to an amount of the agent effective to inhibit activation of a MC.

The term "Siglec-7" also known as AIRM1; CD328; CDw328; D-siglec; QA79; SIGLEC-7; SIGLEC19P; SIGLECP2; p75; and p75/AIRMl refers to a functional, naturally-occurring Siglec-7 polypeptide (e.g., wild-type, splice variant etc.) which is expressed on MCs and other cells such as NK cells and which elicits inhibition of activation of MCs. According to a specific embodiment, the Siglec-7 polypeptide refers to the human protein, such as provided in the following GenBank Numbers NP_001264130, NP_055200 and NP_057627.

Siglec-7 is a type 1 trans-membrane protein characterized by a sialic acid binding N-terminal V-set Ig domain, two C2-set Ig domains and an intracytoplasmic region containing one immune-receptor tyrosine based inhibitory motif (ITIM) and one ITIM-like motif.

As used herein, the terms "agent" and "multivalent agent" refer to a single molecule, or a complex of molecules linked together that have at least two moieties spaced apart from each other in a manner that enables a spatial co-binding and co-activation of at least two receptors being expressed on the same MC. The at least two receptors can be identical, hence the agent will be monospecific and mediate cross-linking. Alternatively, the at least two receptors can be of different structure hence the agent will be at least bi-specific and mediate co-cross-linking. The agent can include for example, but not limited to, an antibody, a small molecule, a ligand, and a combination thereof.

The agents of some embodiment of the invention are provided in an isolated form i.e., isolated from the natural environment e.g., the human body.

As used herein, a "moiety" refers to a functional component capable of binding and activating a receptor on a MC.

Generally, the affinity of the agent (e.g., antibody) is at least 1000 nM, 100 nM, 10 nM or 1 nM.

According to a specific embodiment, the multivalent agent is an agent which binds and activates Siglec-7.

According to a specific embodiment, the multivalent agent is not a monospecific anti-Siglec-7 antibody.

As used herein, "an agent which binds and activates Siglec-7" refers to a molecule having a sufficient binding affinity to Siglec-7 expressed on a MC enough to elicit the transmission of an inhibitory signal leading to inhibition of activation of the MC.

According to specific embodiments, binding of the agent which binds and activates Siglec-7 induces recruitment of intracellular phosphatases.

Non limiting examples of intracellular phosphatases include SHP-1, SHP-2, SHIP-1 and SHIP-2.

According to a specific embodiment, the intracellular phosphatase is SHP-1.

Methods of monitoring activation of Siglec-7 are known in the art and include for example evaluating signal transduction molecules known to be involved in Siglec-7 activation such as recruitment of cytoplasmic phosphatases having a Src homology 2 (SH2) domain such as SHP-1,-2 and SHIP-1,-2 by e.g. co- immunoprecipitation, as well as other methods evaluating activation and/or inhibition of activation of the MC as described hereinabove.

According to an alternative embodiment, there is provided an agent comprising:
(i) a first moiety which binds and activates Siglec-7; and
(ii) a second moiety which binds and activates a MC activating receptor.

The at least two moieties can be linked via any suitable means that does not sterically hinder binding and activation. Non limiting examples of linkers include, an amino acid based linker, a hydrocarbon based linker, cross linker such as 5',5'-dithiobis(2-nitrobenzoic acid) (DNTB), as described [Graziano, R. F. and Guptill, P. (2004) Chemical production of Bispecific antibodies. In: Methods in Molecular Biology. Vol. 283. Edited by C. M. Niemeyer, Human Press Inc., Totowa, NJ.], a linker compound, a carrier, a synthetic spacer, an immobilizing substrate such as beads, (Gly4Ser)3 motif based flexible region, (GGGGS)3 linker with 15 amino acid residues and a modified IgG2b hinge peptide with 22 residues.

According to specific embodiments the at least two moieties are linked covalently although non-covalent interaction is also possible. According to some embodiments binding can be via any one of a cross-linker, a linker compound, a carrier, a synthetic spacer, an immobilizing substrate and a (Gly₄Ser)₃ motif based flexible region.

According to specific embodiments the multivalent agent is monospecific.

As used herein, the term "monospecific" refers to a molecule or a complex of molecules that binds to one type of receptor (i.e. Siglec-7). The moieties of the monospecific agent can be either different (e.g., binding different epitopes on Siglec-7) or identical.

According to a specific embodiment the agent is at least bi-specific.

As used herein, the term "bi-specific" refers to a molecule or a complex of molecules that binds to two types of receptors.

Thus, the multivalent agent may comprise:
(i) a first moiety which binds and activates the Siglec-7; and
(ii) a second moiety which binds and activates a MC activating receptor.

As used herein, the term "activating receptor" refers to a membrane bound or a membrane associated macromolecule or macromolecules complex comprising a ligand-binding region and a cytosolic signaling region, that upon binding of a ligand transmit a signal for activation of the MC. According to a specific embodiment the activating receptor is a human activating receptor.

According to specific embodiments the activating receptor is selected from the group consisting of IgE, FcεRI, c-kit and CD48.

As used herein, the term "c-Kit" also known as KIT, CD117, PBT and SCFR refers to a cell surface receptor tyrosine kinase expressed on MCs that upon binding activates signal transduction molecules that propagate an activation signal in the MC. Signaling through c-Kit is known to be involved in MCs survival, proliferation and differentiation.

As used herein, the term "CD48" also known as BCM1, BLAST, BLAST 1, MEM-102 and SLAMF2 refers to a cell surface GPI anchored receptor expressed on MCs that upon binding activates signal transduction molecules that propagate an activation signal in the MC.

As used herein, the term "IgE" refers to a class of antibodies of the IgE class. Binding to an IgE already bound to a MC by the FcεRI activates signal transduction molecules that propagate an activation signal in the MC.

According to specific embodiments the activating receptor is FcεRI.

As used herein, the term "FcεRI" refers to a receptor complex with high affinity for the Fc region of IgE constitutively expressed on MCs that that upon binding activates signal transduction molecules that propagate an activation signal in the MC.

The αβγ₂ isoform of FcεRI is known to be expressed on MCs whereas, an αγ₂ isoform is known to be expressed also on monocytes, Langerhans cells and DCs. Thus, according to specific embodiments the FcεRI is the tetrameric complex αβγ₂.

According to specific embodiments the agent comprises an antibody or a fragment thereof.

A schematic re-presentation of possible mechanisms of action on tumor MCs induced by activation of Siglec-7 by an antibody is represented in Figure 9.

According to specific embodiments, the multivalent agent comprises an antibody or antibody fragment.

According to an embodiment, the antibody specifically binds at least one epitope of a Siglec-7.

According to specific embodiments the first moiety is an anti-Siglec-7 antibody.

According to specific embodiments the second moiety is an anti MC activating receptor antibody.

The antibodies can be selected from preexisting antibodies (e.g., publicly available hybridomas or commercially available antibodies) or from newly generated antibodies produced according to methods which are well-known in the art and further described hereinbelow. Non limiting examples of commercially available antibodies include anti-human-Siglec-7, clone QA79, can be obtained for example from eBioscience.

The term "antibody" as used in this invention includes intact molecules as well as functional fragments thereof, such as Fab, F(ab')2, Fv or single domain molecules such as VH and VL to an epitope of an antigen that are capable of binding and activating Siglec-7 and/or a MC activating receptor. These functional antibody fragments are defined as follows:
(1) Fab, the fragment which contains a monovalent antigen-binding fragment of an antibody molecule, can be produced by digestion of whole antibody with the enzyme papain to yield an intact light chain and a portion of one heavy chain;
(2) Fab', the fragment of an antibody molecule that can be obtained by treating whole antibody with pepsin, followed by reduction, to yield an intact light chain and a portion of the heavy chain; two Fab' fragments are obtained per antibody molecule;
(3) (Fab')2, the fragment of the antibody that can be obtained by treating whole antibody with the enzyme pepsin without subsequent reduction; F(ab')2 is a dimer of two Fab' fragments held together by two disulfide bonds;
(4) Fv, defined as a genetically engineered fragment containing the variable region of the light chain and the variable region of the heavy chain expressed as two chains;
(5) Single chain antibody ("SCA"), a genetically engineered molecule containing the variable region of the light chain and the variable region of the heavy chain, linked by a suitable polypeptide linker as a genetically fused single chain molecule; and
(6) Single domain antibodies are composed of a single VH or VL domains which exhibit sufficient affinity to the antigen.

Methods of producing polyclonal and monoclonal antibodies as well as fragments thereof are well known in the art (See for example, Harlow and Lane, Antibodies: A Laboratory Manual, Cold Spring Harbor Laboratory, New York, 1988. Antibody fragments according to some embodiments of the invention can be prepared by proteolytic hydrolysis of the antibody or by expression in E. coli or mammalian cells (e.g. Chinese hamster ovary cell culture or other protein expression systems) of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, by Goldenberg, U.S. Pat. Nos. 4,036,945 and 4,331 647. See also Porter, R. R. [Biochem. J. 73: 119-126 (1959)]. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Fv fragments comprise an association of VH and VL chains. This association may be noncovalent, as described in Inbar et al. [Proc. Nat'l Acad. Sci. USA 69:2659-62 (19720]. Alternatively, the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (sFv) are prepared by constructing a structural gene comprising DNA sequences encoding the VH and VL domains connected by an oligonucleotide. The structural gene is inserted into an expression vector, which is subsequently introduced into a host cell such as E. coli. The recombinant host cells synthesize a single polypeptide chain with a linker peptide bridging the two V domains. Methods for producing sFvs are described, for example, by [Whitlow and Filpula, Methods 2: 97-105 (1991); Bird et al., Science 242:423-426 (1988); Pack et al., Bio/Technology 11:1271-77 (1993); and U.S. Pat. No. 4,946,778. Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry [Methods, 2: 106-10 (1991)].

According to specific embodiments, the agent is not a naturally occurring anti-Siglec-7 antibody.

According to specific embodiments, the agent is not an antibody produced by immunization with Siglec-7.

According to specific embodiments, the agent may include molecules having antibody derived regions [such as F(ab') regions linked to each other via a linker/spacer or a recombinant single chain (ScFv)].

According to specific embodiments, the agent is an anti-Siglec-7 antibody that has two F(ab') domains each capable of binding a Siglec-7 receptor. However, it may be that the spacing of the two F(ab') domains in a single antibody is not optimal for binding of two Siglec-7 receptors. Therefore, according to specific embodiments the agent is a synthetic molecule having two F(ab') domains spaced apart by a suitable linker. According to other specific embodiments, the agent contains two or more anti-Siglec-7 receptor antibodies linked or conjugated to each other.

According to specific embodiments, the agent is a bi-specific antibody.

As used herein, the term "bi-specific antibody" or bispecific antibody" refers to an antibody molecule that binds two different antigens one of them is Siglec-7 and the other is a MC activating receptor. The bi-specific antibody can be monovalent or bivalent (or more) for each of the receptor it binds.

Thus, according to specific embodiments the bi-specific antibody is bivalent, trivalent or tetravalent.

According to specific embodiments, each of the binding and activating moeities is selected from any one of a naturally occurring, synthetic or recombinant antibody, single chain Fv (scFv), bi-functional scFv, diabody, F(ab) unit, F(ab') unit, bi-specific F(ab') conjugate, chemically cross-linked bi-functional antibody, linear antibody or a F(ab')2 antigen binding fragment of an antibody.

According to a specific embodiment, the binding and activation moiety is a F(ab') unit.

According to specific embodiments, the bi-specific antibody comprises one arm recognizing Siglec-7 and a second arm recognizing IgE or c-Kit.

Bi-specific antibodies can be produced by many methods known in the art such as those disclosed in US Patent Numbers 4,474,893, 5,959,084, US and 7,235,641, 7,183,076, U.S. Publication Number 20080219980 and International Publication Numbers WO 2010/115589, WO2013150043 and WO2012118903 and include, for example, chemical cross-linking (Brennan, et ai., Science 229,81 (1985); Raso, et ai., J. BioI. Chern. 272, 27623 (1997)), disulfide exchange, production of hybrid-hybridomas (quadromas), by transcription and translation to produce a single polypeptide chain embodying a bi-specific antibody, or by transcription and translation to produce more than one polypeptide chain that can associate covalently to produce a bi-specific antibody.

The contemplated bi-specific antibody can also be made entirely by chemical synthesis.

The bi-specific antibody may comprise two different variable regions, two different constant regions, a variable region and a constant region, or other variations.

The bi-specific antibody may comprise a chemical linking agent covalently incorporated into the bi-specific antibody.

Single chain variable fragments have been connected to each other to form a Bi-specific antibody by various techniques: cross-linking C-terminal cysteine residues, adding naturally associating helices from a four-helix bundle, adding leucine zippers, adding a CH3 domain with either a lmob or hole at the interacting surfaces, or by connecting CHI and CL domains to the respective scFV fragments (Conrath, et at., J. BioI. Chern. 276, 7346 (2001).

Chemically constructed bi-specific antibodies may be prepared by chemically cross-linking heterologous Fab or F(ab')2 fragments by means of chemicals such as heterobifunctional reagent succinimidyl-3-(2-pyridyldithiol)-propionate (SPDP, Pierce Chemicals, Rockford, Ill.).

Oligopeptides and polypeptides may be used for linking two different antibodies or antibody chains together. Oligo- and polypeptides may be synthesized by solution phase or by solid phase techniques. These include processes such as are described in Stewart and Young, Solid Phase Peptide Synthesis, Pierce Chemical Co., Rockford, IL (1984); Bodanszky, The Principles of Peptide Synthesis, 2nd ed., Springer, New York (1993); and Molina, et al., Pept. Res. 9, 151 (1996). For example, an azide process, an acid chloride process, an acid anhydride process, a mixed anhydride process, an active ester process (for example, p-nitrophenyl ester, N-hydroxy-succinimide ester, or cyanomethyl ester), a carbodiimidazole process, an oxidative-reductive process, or a dicyclohexylcarbodiimide (DCCD)/additive process can be used.

According to specific embodiments the agent is a short peptide with one site for each epitope (one for e.g. IgE or c-Kit and one for Siglec-7) or a longer polypeptide with multiple sites for one or both epitopes.

According to specific embodiments, the bi-specific antibody is composed of two antigen-binding domains of antibodies linked or conjugated to each other. Non limiting examples include chemical synthesis such as disclosed for an anti-c-Kit monoclonal Fab that was linked to the monoclonal Fab of CD300a (Bachelet et al. J Immunol. 2008 May 1;180(9):6064-9). Accordingly, two different mAbs are digested with pepsin to achieve 2 different Fab2. The two Fab2 are reduced to Fab and one of them is interacted with TNB. Afterwards they are incubated together and the reduced Fab attacks the TNB and forms the new bi-specific Fab2.

Production of double or triple bi-specific antibodies can be effected for example by activating free carboxylic groups and then the activated bi-specific Fab2 is incubated with another bi-specific Fab2 and covalent bonds are formed between the two to yield a double bi-specific antibody.

Quadromas may be constructed by fusing hybridomas that secrete two different types of antibodies against two different antigens (Milstein and Cuello, Nature 305, 537(1983); Kurokawa et al., Biotechnology 7, 1163 (1989).

Thus, according to specific embodiments, it is possible to obtain a quadroma like construct by fusing together two different hybridomas, for example one anti-c-Kit and the other anti-Siglec-7 to yield a new cell line producing antibody with four arms, 2 against one specificity (i.e. c-Kit) and 2 against the other (i.e. Siglec-7).

Bi-specific antibodies can also be prepared by the transfectoma method (Morrison, Science 229, 1202 (1985).

The invention additionally encompasses bi-specific antibody structures produced within recombinant microbial hosts as described in PCT application WO 93/11161 and Holliger, et al., Proc. NatI. Acad. Sci. USA, 90, 6444 (1993).

Also included are bi-specific linear molecules, such as the so-called "Janusin" structures described by Traunecker, et al., EMBO J. 10,3655 (1991). This can be accomplished by genetically removing the stop codons at the end of a gene encoding a monomeric single-chain antigen-binding protein and inserting a linker and a gene encoding a second single-chain antigen-binding protein (WO 93/11161).

In a further approach, bi-specific antibodies are formed by linking component antibodies to leucine zipper peptides (Kostelny et at., J. ImmunoI. 148, 1547 (1992); de Kruif and Logtenberg, J. BioI. Chern. 271, 7630 (1996)). The leucine zipper occurs in a variety of eukaryotic DNA binding proteins, such as GCN4, *CIEBP,* c-fos gene product (Fos), c-jun gene product (Jun), and c-myc gene product. In these proteins, the leucine zipper creates a dimerization interface wherein proteins containing leucine zippers may form stable homodimers and/or heterodimers. In the formation of bi-specific antibodies, binding fragments of the component antibodies are fused in-frame to first and second leucine zippers.

For clinical applications humanized forms of antibodies (monospecific or at least bispecific) are used. Humanized forms of non-human (e.g., murine) antibodies are chimeric molecules of immunoglobulins, immunoglobulin chains or fragments thereof (such as Fv, Fab, Fab', F(ab').sub.2 or other antigen-binding subsequences of antibodies) which contain minimal sequence derived from non-human immunoglobulin. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers [Jones et al., Nature, 321:522-525 (1986); Riechmann et al., Nature 332:323-327 (1988); Verhoeyen et al., Science, 239:1534-1536 (1988)], by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introduction of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10,: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995).

Alternatively or additionally, at least one of the moieties of the agent comprises a small molecule.

As used herein, the tem "small molecule" refers to a molecule or chemical entity with a low molecular weight, typically smaller than 1000 Daltons.

According to specific embodiments at least one of the moieties in the agent comprises a ligand.

The term "ligand" encompasses both naturally occurring ligands as well as synthetic forms thereof.

As used herein, the phrase "naturally occurring ligand" refers to a substance which is found in nature.

As used herein, the phrase "synthetic ligand" refers to a synthetically induced substance which is not found in nature.

According to specific embodiments the agent comprises a Siglec-7 ligand.

According to specific embodiments the first moiety is a Siglec-7 ligand.

Non limiting examples of Siglec-7 ligand include (2,8)-linked disialic acids and branched alpha 2,6-sialyl residues, such as those displayed by ganglioside GD3 (*N-*acetylneuraminic acid-a2,8-*N*-acetylneuraminic acid-a2,3-galactose-b1,4-glucose), Sia (*N*-acetylneuraminic acid), SiaLe^{x}, (*N*-acetylneuraminic acid-a2,3-galactose-b1,4-(a1,3-fucose)-*N*-acetylglucosamine) and 2,6-SiaLacNAc (*N*-acetylneuraminic acid-a2,6-galactose-b1,4-*N*-acetylglucosamine).

According to specific embodiments the Siglec-7 ligand may be a sialylated glycopolymers such as disclosed in Hudak et al. [Nature Chemical Biology (2014) 10: 69-77]. According to specific embodiments, the second moiety is a MC activating receptor ligand.

Alternatively, the binding moiety for the activating receptor comprises a ligand.

Non limiting examples of IgE ligands include an allergen.

Non limiting examples of FcεRI ligands include IgE.

Non limiting examples of c-Kit ligands include stem cell factor (SCF).

Non limiting examples of CD48 ligands include 2B4 (CD244) and CD2.

According to specific embodiments, the multivalent agent contains at least one ligand either of Siglec-7 and/or a MC activating receptor ligand.

According to specific embodiments, the multivalent agent contains at least two ligands, at least three ligands or at least four ligands.

The agent and methods described herein can be exploited in therapeutic application.

Thus, according to an aspect there is provided a method of treating a MC related cancer in a subject in need thereof, the method comprising administering to the subject a therapeutically effective amount of a multivalent agent which binds and activates Siglec-7, thereby treating the MC related cancer in the subject.

According to another aspect there is provided a multivalent agent which binds and activates Siglec-7, for use in the treatment of a MC related cancer in a subject in need thereof.

According to an aspect, there is provided a method of treating a disease induced by activation of a MC in a subject in need thereof, the method comprising administering to the subject a therapeutically effective amount of a multivalent agent comprising:
(i) a first moiety which binds and activates Siglec-7; and
(ii) a second moiety which binds and activates a MC activating receptor, thereby treating the disease induced by activation of a MC in a subject.

According to another aspect there is provided a multivalent agent comprising:
(i) a first moiety which binds and activates Siglec-7; and
(ii) a second moiety which binds and activates a MC activating receptor,
for use in the treatment of a disease induced by activation of a MC in a subject in need thereof.

As used herein, the phrase "a disease induced by activation of MCs" refers to a disease in which onset or progression of a pathology is attributed at least in part to increase in activation of MCs.

Non-limiting examples of diseases induced by activation of MCs include allergy, asthma, allergic rhinitis, allergic conjunctivitis, atopic dermatitis and atopic eczema, allergic disorders and responses to various allergens, systemic anaphylaxis, anaphylactic response to contrast media, anaphylactic response to muscle relaxants, systemic mastocytosis, mast cell activation syndrome (MAS), morphea/urticaria pigmentosa, mast cell leukemia, atherosclerosis, graft rejection, multiple sclerosis, fibrotic lung diseases, neurofibromatosis, keloids, scleroderma, acute gout, ocular cicatricial pemphigoid, inflammatory disease of the gut such as inflammatory bowel disease, colitis, interstitial cystitis, irritable bowel syndrome, and celiac disease and Crohn's disease, peritoneal adhesions, chronic graft versus host disease (GVHD), extrinsic bronchial asthma, nasal polyposis, Wegener's granulomatosis, intrinsic bronchial asthma, interstitial and other pulmonary diseases, hypersensitivity pneumonitis, allergic bronchopulmonary aspergillosis, sarcoidosis, idiopathic pulmonary fibrosis, toxocariasis, filariasis, schistosomiasis, trichinosis, neoplastic and myeloproliferative diseases, T cell lymphomas, Hodgkin's disease, psoriasis, diabetes mellitus, systemic lupus erythematosus (SLE), Sjogren's syndrome, rheumatoid arthritis, rheumatoid osteoarthritis, polymyositis, autoimmune thyroid disease, autoimmune gastritis and pernicious anemia, and autoimmune hepatitis

According to specific embodiments the disease is selected from the group consisting of allergic disease, inflammatory disease, autoimmune disease, hypersensitivity disease and cancer.

According to specific embodiments the disease is an allergic disease.

According to specific embodiments the disease is asthma.

According to specific embodiments the disease is cancer.

As used herein the terms "cancer" and "mast cell related cancer" refer to a cancerous disease which characterized by the presence of too many MCs in the body for onset and/or progression. Cancerous MCs may be associated with phenotypes such uncontrolled proliferation, loss of specialized functions, immortality, significant metastatic potential, significant increase in antiapoptotic activity, rapid growth and proliferation rate, and certain characteristic morphology and cellular markers. In some circumstances, cancerous MCs will be in the form of a tumor, such cells may exist locally within an animal or circulate in the blood stream as independent cells. It will be appreciated that the term cancer as used herein encompasses all types of MC related cancers, at any stage and in any form.

Non-limiting examples of MC related cancers can be any solid or non-solid MC cancer and/or cancer metastasis, including, but not limited to systemic mastocytosis (SM), mastocytoma, mast cell sarcoma (MCS) and mast cell activation Syndrome (MACS).

As used herein, the term "systemic mastocytosis (SM)" encompasses the 5 categories of SM defined by the World Health Organization according to their location and aggressiveness: cutaneous mastocytosis (CM), indolent SM (ISM), SM with associated clonal hematologic non-MC disease, aggressive SM (ASM), and MC leukemia (MCL). The prognosis of patients with ASM and MCL is poor due to an aggressive nature of the cells and their tendency to detach from the main tumor. Many of these tumors carry mutations in the tyrosine kinase receptor: c-Kit (stem cell factor receptor) that renders it constitutively activated leading to uncontrolled growth of the malignant MCs.

Thus, according to specific embodiments, the cancer is selected from the group consisting of indolent SM (ISM), cutaneous mastocytosis (CM), SM with an associated clonal hematological non-MC-lineage disease (SM-AHNMD), aggressive SM, and mast cell leukemia.

According to specific embodiments the cancer is indolent SM.

The term "mast cell activation Syndrome (MACS)" refers to the recently defined MC related syndrome characterized in 1) two or more symptoms or organ involvement consisted with MC activation, 2) at least one of the MC mediator levels are elevated during acute episode or at baseline levels and 3) patients respond to drugs that block the action of MC mediators or their production. A subtype of MACS, called monoclonal MACS (MMACS/MMAS) can be seen in patients who present 2 or less SM criteria.

As used herein, the term "treating" refers to inhibiting, preventing or arresting the development of a pathology (disease, disorder, or condition e.g., allergy, asthma, mast call related cancer) and/or causing the reduction, remission, or regression of a pathology. Those of skill in the art will understand that various methodologies and assays can be used to assess the development of a pathology, and similarly, various methodologies and assays may be used to assess the reduction, remission or regression of a pathology.

As used herein the phrase "subject in need thereof" refers to a mammalian male or female subject (e.g., human being) who is diagnosed with the disease. Veterinary uses are also contemplated.

The agents of some embodiments of the invention can be administered to an organism *per se,* or in a pharmaceutical composition where it is mixed with suitable carriers or excipients.

Thus, according to another aspect there is provided a pharmaceutical composition comprising, as an active ingredient, a multivalent agent which binds and activates Siglec-7, wherein the multivalent agent is not a monospecific anti-Siglec-7 antibody; and a pharmaceutically acceptable carrier or excipient.

According to another aspect there is provided a pharmaceutical composition comprising, as an active ingredient, an agent comprising:
(i) a first moiety which binds and activates Siglec-7; and
(ii) a second moiety which binds and activates a MC activating receptor; and a pharmaceutically acceptable carrier or excipient.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to any one of the agents which bind and activate Siglec-7, accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition. Suitable routes of administration may, for example, include oral, nasal, tracheal, rectal, transmucosal, especially transnasal, intestinal or parenteral delivery, including intramuscular, subcutaneous and intramedullary injections as well as intrathecal, direct intraventricular, intracardiac, e.g., into the right or left ventricular cavity, into the common coronary artery, intravenous, intraperitoneal, intranasal, or intraocular injections.

Conventional approaches for drug delivery to the central nervous system (CNS) include: neurosurgical strategies (e.g., intracerebral injection or intracerebroventricular infusion); molecular manipulation of the agent (e.g., production of a chimeric fusion protein that comprises a transport peptide that has an affinity for an endothelial cell surface molecule in combination with an agent that is itself incapable of crossing the BBB) in an attempt to exploit one of the endogenous transport pathways of the BBB; pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide). However, each of these strategies has limitations, such as the inherent risks associated with an invasive surgical procedure, a size limitation imposed by a limitation inherent in the endogenous transport systems, potentially undesirable biological side effects associated with the systemic administration of a chimeric molecule comprised of a carrier motif that could be active outside of the CNS, and the possible risk of brain damage within regions of the brain where the BBB is disrupted, which renders it a suboptimal delivery method.

Alternately, one may administer the pharmaceutical composition in a local rather than systemic manner, for example, via injection of the pharmaceutical composition directly into a tissue region of a patient.

Pharmaceutical compositions of some embodiments of the invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with some embodiments of the invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by oral and/or nasal inhalation, the active ingredients for use according to some embodiments of the invention are conveniently delivered in the form of an aerosol spray presentation from a pressurized pack, metered dose inhaler or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch. For administration by oral inhalation the active ingredients for use according to some embodiments of the invention can also be delivered by a dry powder inhaler.

The pharmaceutical composition described herein may be formulated for parenteral administration, e.g., by bolus injection or continuousinfusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of some embodiments of the invention may also be formulated in rectal compositions such as suppositories or retention enemas, using, e.g., conventional suppository bases such as cocoa butter or other glycerides.

Methods of administering an active agent into a skin are known in the art and include, for example, intradermal injections, gels, liquid sprays and patches which comprise the active agent and which are applied on the outer surface of the skin.

According to some embodiments of the invention, administration of the active agent into the skin of the subject is performed topically (on the skin).

According to some embodiments of the invention, administration of the active agent into the skin of the subject is performed non-invasively, e.g., using a gel, a liquid spray or a patch comprising the active ingredient, which are applied onto the skin of the subject.

There are two main types of skin patches which can be used to administer the agents into the skin of a subject. These are the reservoir type patch and the matrix type patch. The reservoir patch usually contains a structure filled with a solid drug (active agent) and a dilute solution, or a highly concentrated drug solution within a polymer matrix and is surrounded by a film or membrane of rate-controlling material. The matrix patch contains a drug and a polymer which form a homogenous system from which the drug is released by diffusion into the external environment. It should be noted that as the release continues, its rate in the matrix type patch usually decreases since the active agent has a progressively longer distance and therefore requires a longer diffusion time to release. For further details and examples of transdermal drug delivery see Prausnitz MR., et al., 2004. Nature Reviews, 3:115-124; Scheindlin S., 2004. Transdermal drug delivery: Past, present, future. Molecular Interventions. Vol. 4:308-312; Prausnitz MR and Langer R., 2008, Nature Biotechnology. 26:1261-1268; Tanner T, and Marks R, 2008, Delivery drugs by transdermal route: review and comment. Skin Research and Technology, 14: 249-260. A non-limiting example of an epicutaneous drug delivery patch, which can be used to administer the agent into the skin according to the teachings of the invention, is described in Senti G., et al., 2009, J Allergy Clin Immunol. Sep 4. [Epub ahead of print]. According to some embodiments of the invention, administering the agent to the skin is performed using a reservoir type patch.

According to some embodiments of the invention, administering the agent to the skin is effected on an intact skin (e.g., a skin which has not been breached, peeled or physically/chemically permeabilized).

For example, administering into an intact skin can be performed using an occlusive patch with semi-solid reservoir and a plastic backing adhesive contour and protective removable cover.

A semi-solid reservoir can be any gel, cream, ointment, emulsion, suspension, microparticles, using various excipients such as fats, oils (e.g., mineral oil, vaseline, vegetable oil or silicon oil), polymers, gelling agent, suspending agent, stabilizers, hydrophilic solvents, Propylene glycol, polyethylene glycols, stabilizing surfactants, colloids etc. and their combinations.

It should be noted that in order to increase delivery of the active agent into the skin, the active agent can be formulated with various vehicles designed to increase delivery to the epidermis or the dermis layers. Such vehicles include, but are not limited to liposomes, dendrimers, noisome, transfersome, microemulsion and solid lipid nanoparticles (for further details see Cevc, G. Transfersomes, liposomes and other lipid suspensions on the skin: permeation enhancement, vesicle penetration, and transdermal drug delivery. Crit. Rev. Ther. Drug Carrier Syst. 13, 257-388 (1996), Kogan A, Garti N. Microemulsions as transdermal drug delivery vehicles. Adv Colloid Interface Sci 2006; 123-126:3 69-385). In addition, the active agent can be mixed with chemical enhancers such as sulphoxides, azones, glycols, alkanols and terpenes which enhance delivery of active agents into the skin (for further details see Karande P, Jain A, Ergun K, Kispersky V, Mitragotri S. Design principles of chemical penetration enhancers for transdermal drug delivery. Proc Natl Acad Sci U S A 2005;102:4688-4693; Williams AC, Barry BW. Penetration enhancers. Adv Drug Deliv Rev 2004;56:603-618; and Smith, EW.; Maibach, HI., editors. Boca Raton, FL: Taylor and Francis Group; 2006. Percutaneous Penetration Enhancers). The patch may include the agent formulated within an emulsion designed to facilitate permeabilization of drugs to the epidermis or the dermis. For example, the patch may comprise the agent within an oil-in-glycerin emulsion, which is designed to facilitate permeabilization of the agent through the stratum-corneum and into the dermis. A non-limiting example of an oil-in-glycerin emulsion suitable for delivery through the stratum-corneum into the dermis is described in US Patent Application No. 20040067244. Such an oil-in-glycerin emulsion exhibits a mean droplet size below one micron, and comprises a continuous glycerin phase; at least one vegetable oil comprising an internal phase; at least one emulsifying stabilizer; and at least one bioactive compound comprising at least one hydrophobic, moiety within its structure, wherein the composition facilitates permeabilization of the bioactive compound through the stratum-corneum and into the dermis.

According to some embodiments of the invention, administering the agent to the skin is effected on a breached skin [e.g., a skin that has been permeabilized (e.g., ruptured) with an external object and the like].

According to some embodiments of the invention, breaching of the skin is effected temporarily (e.g., performed for a pre-determined short period) and is designed to enable better permeabilization of the active ingredient into the skin.

Breaching of the skin can be performed, for example, by introducing micro-holes (e.g., microchannels) in the outer layer of the skin. Such microchannels can be formed using for example, the Radio-Frequency (RF)-Microchannel™ (TransPharma Medical™ Ltd.) technology [Hypertext Transfer Protocol ://World Wide Web (dot) transpharma-medical (dot) com/technology_rf (dot) html].

Additionally or alternatively, delivery of the active agent from the patch to the epidermis layer of the skin can be enhanced using physical enhancers known in the art such as ultrasound, ionophoresis, electroporation, magnetophoresis, microneedle and continuous mixing [see e.g., Rizwan M, Aqil M, Talegaonkar S, Azeem A, Sultana Y, Ali A. Enhanced transdermal drug delivery techniques: an extensive review of patents. Recent Pat Drug Deliv Formul. 2009;3(2):105-24]. According to some embodiments of the invention, administering the agent is performed by an intradermal injection.

The agent can be administered into the dermal layer of the skin of the subject by an intradermal injection as described for the Mantoux C (1908) test. Briefly, the agent can be injected intracutaneously (using for example, a 0.5-ml or 1.0 ml tuberculin syringe through a 26-gauge or 27-gauge needle). The syringe can be placed at an angle of 45 degrees to the skin, and the bevel of the needle is angled downward, facing the skin, and penetrating entirely but not deeper than the superficial layers of the skin. A volume of approximately 0.01 to 0.05 ml (e.g., about 0.02 ml) is gently injected to produce a small superficial bleb (Middleton's Allergy principles&practice, 6th edition 2003).

According to some embodiments of the invention, administering the agent is performed using a liquid spray (e.g., a spray which includes the agent in a pre-determined concentration and dosage).

According to some embodiments of the invention, administering the agent is performed using a gel (e.g., a gel which includes the agent in a pre-determined concentration and dosage).

For example, for administration using a gel or a spray, a predefined area for administration of the agent is selected and optionally bounded using an accessory equipment (see e.g., Hypertext Transfer Protocol://World Wide Web (dot) truetest (dot) com).

According to some embodiments of the invention, administering the agent to the skin is effected such that the required agent dose is delivered to the skin epidermis and/or dermis layers within a short time, mimicking the effect of an injection into the epidermis or dermis layers.

Pharmaceutical compositions suitable for use in context of some embodiments of the invention include compositions wherein the active ingredients are contained in an amount effective to achieve the intended purpose.

As used herein, a "therapeutically effective amount" refers to an amount of the agent effective to prevent, alleviate or ameliorate symptoms of a disorder (e.g., allergic disease, inflammatory disease, cancer) or prolong the survival of the subject being treated.

Determination of an effective amount and a therapeutically effective amount is well within the capability of those skilled in the art, especially in light of the detailed disclosure provided hereinbelow. For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide that the levels of the active ingredient are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of some embodiments of the invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as is further detailed above.

It is also contemplated that the agent is administered alone or in combination with other therapeutic such as, but not limited to, an anti inflammatory agent, an anti allergic agent, a chemotherapeutic agent, an immunosuppressive agent, and an anti-malarial agent.

Non limiting examples of therapeutic agents that can be used include corticosteroids, glucocorticoids, soluble tumor necrosis factor receptor, antibodies against tumor necrosis factor, granulocyte colony stimulating factor, interferon-y, picetannol, benzamidines, tenidap, tiacrilast, disodium cromoglycate, Iodoxamide ethyl, Iodoxamide tromethamine, quercetin, antihistamines such as diphenhydramine and theophylline, serine protease inhibitors such as α-1-protease inhibitor, metalloprotease inhibitors, lisofylline, benzamidine, amiloride, bis-amidines such as pentamidine and bis(5-amidino-2-benzimidazolyl)methane, FK-506, methotrexate, vincristine, and cyclophosphamide.

According to specific embodiments, the agent is administered in combination with an agent that stimulates expression of an inhibiting receptor or an activating receptor.

The finding that Siglec-7 is expressed on MCs further suggests its use in the identification and characterization of MCs.

Thus, according to another aspect, there is provided a method of identifying a MC, the method comprising detecting presence or level of Siglec-7 in cells of a biological sample, wherein presence of Siglec-7 or level above a predetermined threshold is indicative of a MC in a biological sample.

According to specific embodiments, the method further comprises analyzing a presence of a MC marker which is different from Siglec-7 in the cells for corroborating the detection.

According to specific embodiments the MC marker is selected from the group consisting of a cell surface marker (e.g., as indicated hereinabove e.g., FcεRI, CD48 and c-kit), a morphological marker (e.g., granulocytes) and an activity marker (e.g., functional mediator release).

Methods of analyzing gene expression are well known in the art and include, but are not limited to FACS, Western blotting, PCR, RT-PCR, Northern blotting and the like.

As used herein the term "about" refers to ± 10 %

The terms "comprises", "comprising", "includes", "including", "having" and their conjugates mean "including but not limited to".

The term "consisting of' means "including and limited to".

The term "consisting essentially of' means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

It is appreciated that certain features, which are, for clarity, described in the context of separate embodiments, may also be provided in combination in a single embodiment. Conversely, various features, which are, for brevity, described in the context of a single embodiment, may also be provided separately or in any suitable subcombination or as suitable in any other described embodiment. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996).

### MATERIALS AND METHODS

***Cells* -** Human cord-blood mast cells (CBMCs) were obtained as described (Piliponsky et al. Blood (2003) 1101:1898-1904). Briefly, human umbilical cord-blood was obtained from normal births. Mononuclear cells were enriched by Ficoll-density centrifugation and grown in MEM-α medium supplemented with SCF (SOBI, Stockholm, Sweden), IL-6 (Peprotech, Rocky Hill, NJ) and PGE₂ (SIGMA, St. Louis, MO). Following 8 weeks of culturing, CBMCs were evaluated by acidic toluidine blue staining and cultures with > 90 % mature mast cells (MCs) were used. Tissue-residing mature MCs were obtained from infant foreskin as previously reported (Benyon et al. J. Immunol (1987) 138:861-7) with the addition of MCs isolation step, following tissue digestion to a single cell suspension, using the CD117 MicroBead Kit (Miltenyi Biotec, Bergisch Gladbach, Germany) according to manufacturer's instructions. Human peripheral mononuclear cells (PBMCs) were enriched from peripheral blood samples by Ficoll-density centrifugation. HMC1 cells and LAD2 cells were obtained as described [Butterfield et al. Leuk Res. (1988) 12(4):345-55; and Kirshenbaum et al. Leuk Res. (2003) 27(8):677-82, respectively]. Primary NK cells and NK cell lines were obtained as described (Tsukerman P et al, Eur J Immunol. 44(5):1517-25 and Mandelboim O et al, J Exp Med. 184(3):913-22, respectivly).

***Cell treatments*** - CBMCs were sensitized for 4 days with human IgE (300 ng / ml, Calbiochem, EMD Millipore, Billerica, MA) in the presence of human IL-4 (10 ng / ml, Peprotech). On day 5, cells were washed and blocked with 5 % goat serum in PBS for 10 minutes on ice. Thereafter, cells were incubated in Tyrode's buffer (Bachelet et al. J. Immunol. (2005) 175:7989-95) containing the indicated concentrations of mouse anti-human-IgE (clone 4C3, Serotec, Oxford, UK) together with mouse anti-human-Siglec-7 (clone eBioQA79, eBioscience, San Diego, CA) or matched isotype control (mIgG1, eBioscience) for 30 minutes on ice. Thereafter, cells were centrifuged and activated for 30 minutes in 37 °C, by re-suspension in Tyrode's buffer containing 0.9 mM MgCl₂, 1.8 mM CaCl₂ and the co-cross linker F(ab')₂ goat anti-mouse IgG (10 mg / ml, Jackson ImmunoResearch Laboratories, West Grove, PA).

For the non-co-cross-linked assay, CBMCs were sensitized and blocked under the same conditions as described hereinabove, but pre-incubated only with mouse anti-Siglec-7 or mIgG1. Thereafter, goat anti-mouse cross linker was added for cross-linking of anti-Siglec-7 or mIgG1 mAb and cells were activated with rabbit anti-human IgE polyclonal (pAb) (1.5 µg / ml, Dako, Glostrup, Denmark).

To test GM-CSF release, CBMCs were treated and activated in the same way, however for only 24 hours in CBMC medium.

Following treatment, supernatants and cell pellets were collected and kept frozen for further analysis.

To detect p-SHP-1 and general tyrosine phosphorylation, 6 x 10⁶ CBMCs / sample were incubated with 1 mM sodium orthovanadate for 10 minutes in 37 °C.

***Functional Mediator Release assays* -** Supernatants and cell pellets were analyzed for the release of tryptase and β-hexosaminidase (β-hex) in colorimetric enzymatic assays [Bachelet et al. J. Immunol. (2005) 175:7989-95] and the release of histamine in a fluorometric assay [Gibbs et al. Clin Exp Allergy (2008) 38:480-5]. Percentage release was calculated by dividing the amount of the mediator detected in the supernatant by the total amount present in the cell pellet and supernatant and multiplying by 100. Supernatants were analyzed for PGD₂ and GM-CSF release by commercial ELISA kits (Cayman chemicals, Ann Arbor, MI and Peprotech, respectively), according to manufacturers' instructions. CBMCs functional assays were performed in triplicates.

***Flow cytometry analysis* -** For detection of Siglec-7 expression in different MC types, cells were blocked with 5 % goat serum and resuspended in flow cytometry (FC) buffer (0.5 % BSA and 0.05 % sodium azide in PBS) containing anti-Siglec-7 (clone QA79, 5 µg / ml) or isotype control mAb (eBiosience). Thereafter, cells were centrifuged, washed and incubated with FC buffer containing FITC-conjugated F(ab')2 goat anti-mouse IgG (1:200, Jackson ImmunoResearch Laboratories).

For the detection of Siglec-7 expression level on NK cells or monocytes, (Figure E1A), freshly isolated peripheral blood mononuclear cells (PBMCs), or IL-2 supplemented NK cells (NK line) were blocked and stained for Siglec-7 as described hereinabove using Alexa Fluor 647-conjugated goat anti-mouse as a secondary Ab (Invitrogen). Thereafter, cells were centrifuged and incubated with FITC-conjugated mouse anti-human CD3 and PE-conjugated mouse anti-human CD56 for the detection of Siglec-7 on NK cells (CD56 positive, CD3 negative) or with FITC-conjugated mouse anti-human CD14 for monocytes. The relevant isotype matched controls were used (all from BioLegend, San Diego, CA).

Following staining, all cells were washed once with FC buffer and analyzed by FACSCalibur equipped with CellQuest software (Becton Dickinson Immunocytometry Systems, San Jose, CA).

MC tumor lines were cultured in 96 wells flat-bottom plates pre-coated with different concentrations of mouse anti-human Siglec-7 mAb (QA79, eBioscience), isotype matched mIgG1 (eBioscience), mouse anti-human Siglec-7 hybridoma's sup (QA79, Vitale C et al, Proc Natl Acad Sci USA. 98(10):5764-9) or non-relevant hybridoma's sup (MA152, Vitale M et al, Eur J Immunol. 31(1):233-42) for 30 minutes on ice. Cells were washed, resuspended in the appropriate medium and transferred to 96 wells flat-bottom plates pre-coated overnight with 10 µg / ml cross linker F(ab')2 goat anti-mouse (Jackson ImmunoResearch Laboratories) or without the cross-linker and incubated for 5 days in 37 °C, at 5 % CO2. Following incubation, cells were harvested, washed and stained with propidium iodide (PI) and analyzed by FACSCalibur equipped with CellQuest software.

***Immunoprecipitation (IP) and Western blot (WB)* -** 6 x 10⁶ CBMCs / sample were lysed with 0.5 % NP-40 lysis buffer [Mizrahi et al. PLoS One (2007) 9:e818], pre-cleared and washed extensively with 0.1 % TBST buffer (0.1 % v / v Tween® 20 in TBS). Thereafter, proteins were co-immunoprecipitated (IP) using protein A/G beads (Santa Cruz Biotechnology, Dallas, Texas) conjugated to Siglec-7 (clone QA79, eBioscience) or mIgG1 isotype control (eBioscience). Immunoprecipitated proteins were resolved by a reducing SDS-PAGE, transferred to a nitrocellulose membrane and blocked with BSA. For the detection of Siglec-7, general phosphotyrosine and phospho-SHP-1, membranes were incubated with pAb goat anti-human Siglec-7 (R&D systems, Minneapolis, MN), mouse anti phosphotyrosine (Santa Cruz) and pAb rabbit anti-p-SHP-1 (ECM Biosciences, Versailles, KY), respectively, followed by the relevant horse-radish peroxidase (HRP)-conjugated secondary Ab.

For the comparison of Siglec-7 content in CBMCs, 100 ng / ml of either anti-Siglec-7 or isotype control in PBS were added to a 96-well ELISA plate and incubated overnight at 4 °C. After removal of unbound Ab, the plate was blocked with 2 % BSA in PBS for 2 hours before washing twice in 0.1 % TBST. 1 x 10⁶ CBMCs / sample were lysed with 0.5 % NP-40 lysis buffer [Mizrahi et al. PLoS One (2007) 9:e818] and lysates were heated at 95 °C for 4 minutes and then added to the appropriate wells and incubated for 3 hours while rocking at room temperature. The plate was then washed 3 times with TBST and 20 µl of glycine buffer (pH 2.0) was added. Following 5 min, proteins were transferred to vials containing 20 µl of Laemmli reagent and then analyzed by SDS-PAGE and WB using anti-Siglec-7 (eBioscience) followed by the relevant HRP-conjugated secondary Ab.

***Immunofluorescence* -** CBMCs, LAD-2 and HMC-1.1 cells were adhered to poly lysine coated slides and fixed with 4 % paraformaldehyde (PFA). Thereafter, the cells were washed and incubated with mouse anti-human Siglec-7 antibody (clone QA79, eBioscience), followed by Alexa Fluor 647-conjugated goat anti-mouse antibody (1:50, Life Technologies, Carlsbad, CA). Immunostained cells were mounted with aqueous mounting medium (Southern Biotech, Birmingham, AL). Analysis was performed using a Zeiss LSM 710 Axio Observer Z1 confocal laser-scanning microscope, equipped with LSM ZEN software (Zeiss, DE, Göttingen, Germany).

***BRDU Assay* -** MC tumor lines were incubated with different concentrations of mouse anti-human Siglec-7 (eBioscience) mAb or 5 µg / ml of control mIgG1 (R&D) for 30 minutes on ice. Cells were washed, resuspended in the appropriate medium and transferred to plates pre-coated with F(ab')2 goat anti-mouse (Jackson ImmunoResearch Laboratories, West Grove, PA) and incubated for 5 days at 37 °C, 5 % CO2. After 4 days, BrdU reagent (Millipore, Billerica, MA) was added for 24 hours. At day 5 cells were harvested and the level of BrdU uptake was determined by a colorimetric assay (Millipore, Billerica, MA) according to manufacturer's instructions. Results are presented after subtraction of the readings from untreated cells with no BrdU added to them.

***Statistical analysis* -** Statistical significance was evaluated by one-tailed and equal variance Student's t-test. P values are indicated in the figure legends.

### EXAMPLE 1

### EXPRESSION OF SIGLEC-7 ON MAST CELLS

Siglec-7 expression was analyzed by flow cytometry and immunofluorescence staining. Siglec-7 was found to be expressed on the surface of normal human mast cells (MCs) derived from cord blood precursors (CBMCs), isolated human foreskin MCs and neoplastic MC lines such as HMC-1, HMC-1.1 and LAD (see Figures 1A-B and 2A). Interestingly, Siglec-7 was expressed in immature CBMCs as early as 4 weeks in culture (data not shown) and this level of expression was maintained thereafter throughout the 8 weeks maturation period.

Siglec-7, originally identified on NK cells as a 75-kDa protein, consists of a 46-kDa peptide backbone modified by extensive glycosylation and therefore may range in size from 46 to at least 75 kDa. To verify the form(s) of Siglec-7 expressed in MCs, Siglec-7 was immunoprecipitated and resolved by a reducing SDS-PAGE, followed by Western blot analysis. As can be seen in Figures 1C and 2B, CBMC-associated Siglec-7 migrated as a 75-kDa protein, indicating high level of glycosylation. This band is specific for Siglec-7 since it was not immunoprecipitated by the mIgG1 isotype control.

### EXAMPLE 2

### CO-CROSS-LINKING OF SIGLEC-7 WITH FcεRI INHIBITS MAST CELLS DEGRANULATION

Degranulation is the amount of mediators released by MCs, which is indicative of the level of MCs activation. The present inventors tested the ability of Siglec-7 to inhibit MCs degranulation induced by FcεRI (IgE receptor)-dependent stimulation using an activating anti-Siglec-7 mAb. As inhibitory receptors (IR) were previously shown to optimally inhibit FcεRI activation-mediated tyrosine kinase signaling cascades by co-cross-linking both the IR and the FcεRI, IgE-sensitized CBMCs were incubated with mouse anti-human IgE mAb with or without mouse antihuman Siglec-7 mAb or its isotype control antibody (mIgG1) at various concentrations, and then added a goat F(ab')2 anti-mouse IgG antibody to co-cross-link and activate Siglec-7 and FcεRI.

One group of mediators released by activated MCs are preformed mediators stored in granules. Two representative of this group are the enzymes tryptase and β-hexosaminidase. As demonstrated in Figures 3A-B, co-cross linking Siglec-7 to the activation signal of FcεRI significantly reduced the secretion of tryptase and β-hexosaminidase in a dose dependent manner.

In order to verify that Siglec-7 mediated inhibition is not specific to one group of mediators released by MCs but rather a more general one, the ability of Siglec-7 to inhibit the release of mediators belonging to the two other main groups of mediators: newly synthesized mediators, and cytokines was tested. As demonstrated in Figures 3C and 3D, co-cross linking Siglec-7 to the activation signal of FcεRI significantly reduced in a dose dependent manner the secretion of the de-novo synthesized lipid mediator PGD2 which belongs to the arachidonic acid metabolites; and of GM-CSF which belongs to the cytokines group that are synthesized and released after activation of MCs.

These results suggest that Siglec-7 functions as an inhibitory receptor on normal human MCs capable of inhibiting the release of mediators belonging to all three major groups of mediators released by activated MC.

To test whether co-cross-linking Siglec-7 with FcεRI is required for its inhibitory function in MCs, IgE-sensitized CBMCs were pre-incubated with mouse anti-Siglec-7 mAb or its isotype control, followed by simultaneously cross-linking anti-Siglec-7 mAb (using a goat F [ab']2 anti-mouse IgG antibody) and activating FcεRI using a polyclonal rabbit antihuman IgE, which could not be cross-linked by the F(ab')2 anti-mouse IgG. Cross-linking Siglec-7 alone did not inhibit FcεRI-induced degranulation (Figure 4), demonstrating that Siglec-7 must be coupled to FcεRI by co-cross-linking to effectively inhibit mediator release from CBMCs.

It is possible that the requirement of Siglec-7 to co-cross-link with FcεRI to inhibit CBMCs activation *in-vitro* is also true *in vivo.* On tissue-resident MCs in vivo, ligated FcεRI (and potentially other activating receptors) and Siglec-7 (and other IRs) likely co-migrate to specific membrane sub-locations where mutual activation and inhibition can occur. In addition, it is possible that intercellular interactions that occur during allergic inflammation can induce robust Siglec-7 clustering strong enough to induce self-activation without the requirement for co-cross-linking.

In order to elucidate the signaling molecules mediating the inhibitory effect of Siglec-7, co- immunoprecipitation of Siglec-7 and SHP-1 [which was previously found to participate in Siglec-7 function in NK cells (Falco et al. J. Exp, Med. (1999) 274: 34089-95] from CMBCs was determined. The results suggested that tyrosine-phosphorylated SHP-1 co- immnunoprecipitated with Siglec-7 in CBMCs (Figure 5). However, although treatment with orthovanadate, a tyrosine phosphatase inhibitor, significantly increased Siglec-7 tyrosine phosphorylation (Figure 5, p-Tyr 75-kDa band), the amount of phosphorylated SHP-1 co- immunoprecipitated with Siglec-7 increased only slightly in CBMCs, indicating that SHP-1 might not be the main signal transduction molecule responsible for mediating the signals downstream of activated Siglec-7 in CBMCs.

### EXAMPLE 3

### CROSS-LINKING OF SIGLEC-7 INDUCES TUMORIGENIC MAST CELLS' DEATH

In order to test whether the cross-linking of Siglec-7 on tumorigenic mast cell lines, such as HMC-1 and LAD-2, would decrease their viability, a propidium iodide staining assay was done. As shown in Figure 6A, cross-linking of Siglec-7 caused massive death of HMC-1 cells treated with anti-Siglec-7 mAb, compared to cells treated with isotype control mAb. Thus, for example, 32.5 % of the cells were PI positive when cells were incubated with 10 µg/ml anti-Siglec-7 compared to only 2.5 % of the cells incubated with 10 µg / ml control antibody. This effect was also evident in LAD-2 cells (Figure 6B) with 86.5 % PI positive cells after incubation with anti-Siglec-7 mAb as compared to 18.6 % after incubation with the isotype control. Interestingly, cross-linking of Siglec-7 did not affect the survival of normal MCs (data not shown).

The experiment was repeated with sups of hybridomas in order to neutralize the possible affect of the preservative martial. As shown in Figure 7A, the cross-linking of Siglec-7 with the sup of the anti-Siglec-7 hybridoma (QA79) and plate-adherent goat anti-mouse increased the mortality of HMC-1.1 cells compared to cells incubated with non-relevant sup hybridoma (NA152). This effect was dose dependent and similar to the effect induced by the pure mAb. These results confirmed that Siglec-7 mAb is directly responsible to the increased mortality of HMC-1.1.

Furthermore, cross-linking of Siglec-7 with anti-Siglec-7 hybridoma sup (QA79) without the additional plate-adherent cross-linker antibody is sufficient to induce the inhibitory effect of Siglec-7 on HMC-1.1 cells. As shown in Figure 7B, cross-linking of Siglec-7 with sup of the anti-Siglec-7 hybridoma (QA79) induced cells death of HMC-1.1 cells similar to the sup of the anti-Siglec-7 hybridoma (QA79) and a cross-linker antibody.

Another important aspect of tumorigenic cells is their high rate of proliferation. Thus, cross-linking of Siglec-7 was tested for its ability to inhibit HMC-1.1 proliferation. Indeed, cross-linking of Siglec-7 with 5 and 1 µg / ml mouse anti-human Siglec-7 antibody reduced their proliferation as measured by BrdU incorporation as compared to cells treated with control antibody (Figure 8).

Taken together, Siglec-7 is expressed in normal human CBMCs, isolated human foreskin MCs and neoplastic MC lines such as HMC-1, HMC-1.1 and LAD. Co-cross-linking of the inhibitory receptor Siglec-7 with the activation receptor FcεRI inhibits degranulation of normal MCs without reducing their viability. In addition, cross-linking of Siglec-7 increases the mortality of transformed MCs and inhibits their proliferation.

## Claims

1. A multivalent monospecific antibody or fragment thereof which binds and activates Siglec-7, for use in the treatment of a mast cell related cancer in a subject in need thereof.

2. The antibody or fragment thereof for use according to claim 1, wherein said cancer is selected from the group consisting of systemic mastocytosis (SM), mastocytoma, mast cell sarcoma (MCS) and mast cell activation Syndrome (MACS).

3. The antibody or fragment thereof for use according to claim 1, wherein said cancer is selected from the group consisting of indolent SM (ISM), cutaneous mastocytosis (CM), SM with an associated clonal hematological non-MC-lineage disease (SM-AHNMD), aggressive SM, and mast cell leukemia.

4. The antibody or fragment thereof for use according to claim 1, wherein said cancer is indolent SM.

5. A method of identifying a mast cell, the method comprising detecting presence or level of Siglec-7 in cells of a biological sample, wherein presence of Siglec-7 or level above a predetermined threshold is indicative of a mast cell in a biological sample.

## Patentansprüche

1. Multivalenter monospezifischer Antikörper oder Fragment davon, der bzw. das Siglec-7 bindet und aktiviert, zur Verwendung bei der Behandlung eines mastzellenbezogenen Krebses bei einem Individuum, das diese benötigt.

2. Antikörper oder Fragment davon zur Verwendung nach Anspruch 1, wobei der Krebs ausgewählt ist aus der Gruppe bestehend aus systemischer Mastozytose (SM), Mastozytom, Mastzellsarkom (MCS) und Mastzellaktivierungssyndrom (MACS).

3. Antikörper oder Fragment davon zur Verwendung nach Anspruch 1, wobei der Krebs ausgewählt ist aus der Gruppe bestehend aus indolenter SM (ISM), kutaner Mastozytose (CM), SM mit einer assoziierten klonalen hämatologischen nicht die MC-Abstammungslinie betreffenden Krankheit (SM-AHNMD), aggressiver SM und Mastzellenleukämie.

4. Antikörper oder Fragment davon zur Verwendung nach Anspruch 1, wobei der Krebs indolente SM ist.

5. Verfahren zum Erkennen einer Mastzelle, wobei das Verfahren das Detektieren des Vorhandenseins oder der Menge von Siglec-7 in Zellen einer biologischen Probe umfasst, wobei das Vorhandensein von Siglec-7 oder einer Menge oberhalb eines vorab festgelegten Schwellenwertes eine Mastzelle in einer biologischen Probe anzeigt.

## Revendications

1. Anticorps monospécifique multivalent ou fragment de celui-ci qui se lie au Siglec-7 et active ce dernier, pour une utilisation dans le traitement d'un cancer lié aux mastocytes chez un sujet en ayant besoin.

2. Anticorps ou fragment de celui-ci pour utilisation selon la revendication 1, ledit cancer étant choisi dans le groupe constitué par une mastocytose systémique (MS), un mastocytome, un sarcome mastocytaire (SM) et le syndrome d'activation mastocytaire (SAMA).

3. Anticorps ou fragment de celui-ci pour une utilisation selon la revendication 1, ledit cancer étant choisi dans le groupe constitué par une MS indolente (MSI), une mastocytose cutanée (MC), une MS associée à une hémopathie clonale non mastocytaire (MS-AHNMD), une MS agressive et une leucémie mastocytaire.

4. Anticorps ou fragment de celui-ci pour une utilisation selon la revendication 1, dans lequel ledit cancer est une MS indolente.

5. Méthode d'identification d'un mastocyte, la méthode comprenant la détection de la présence ou du taux de Siglec-7 dans les cellules d'un échantillon biologique, la présence de Siglec-7 ou un taux supérieur à un seuil prédéterminé étant indicatif d'un mastocyte dans un échantillon biologique.
